(19)

Europäisches
Patentamt
European
Patent Office
Office européen
des brevets

(11) **EP 2 778 990 A2**

(12) **EUROPEAN PATENT APPLICATION**

(43) Date of publication:
**17.09.2014 Bulletin 2014/38**

(51) Int Cl.:
**G06F 17/50** [(2006.01)]

(21) Application number: **14153898.3**

(22) Date of filing: **05.02.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **18.02.2013 GB 201302743**

(71) Applicant: **Rolls-Royce plc**
**London SW1E 6AT (GB)**

(72) Inventors:
• **Conduit, Bryce**
**Fareham, GU9 9JU (GB)**
• **Conduit, Gareth**
**Cambridge, CB2 1TA (GB)**

(74) Representative: **Gee, Philip David et al**
**Rolls-Royce plc**
**Intellectual Property**
**SinB-38, PO Box 31**
**Derby**
**Derbyshire DE24 8BJ (GB)**

(54) **Method and system for designing a material**

(57)    A method of designing a material by optimising values for a plurality of design variables is provided. The method including the steps of:
(i) providing one or more property models, the $i^{th}$ property model (where i is an integer from 1 to $n_{prop}$, the number of property models) producing a prediction $P_i$ for a value of a respective property of the material as a function of the design variables, and further producing a value for the uncertainty $\sigma_i$ in the prediction $P_i$,
(ii) for each property, setting a specification target $T_{0,i}$ for a desired value of the property and a probability $p_i$ for

that specification target $T_{0,i}$ to be met or exceeded,
(iii) for each property, determining a probabilistic target $T_i$ for a value of the property, the probabilistic target $T_i$ being based on the specification target $T_{0,i}$ and the probability $p_i$, and further defining a merit index factor $G_i$ based on the degree to which a given prediction $P_i$ satisfies the probabilistic target $T_i$,
(iv) constructing an overall merit factor G from the individual merit index factors $G_i$ of the properties, and
(v) determining a set of optimal design variables that optimise the overall merit factor G.

Fig. 1

EP 2 778 990 A2

**Description**

Field of the Invention

The present invention relates to a method and a system for designing a material, such as an alloy.

Background of the Invention

When designing new materials in complex material systems, such as nickel-based superalloys, there may be ten or more constituent elements whose relative amounts must be determined. Changing the material composition can affect properties such as strength, creep resistance, and oxidation and corrosion resistance. In addition, one must determine the optimum processing conditions such as heat treatment times and temperatures that can have a profound effect on microstructure and material performance.

Thus, due to the number of possible design variables, historically material design has tended to proceed by a trial-and-error process.

However, with improvements in computational power, the potential exists to put material design on a more systematic footing. For example, R.C. Reed, T. Tao and N. Warnken, *Materials-By-Design: Application to nickel-based single crystal superalloys,* Acta Materialia **57,** 5898 (2009) propose a systematic design approach for nickel-based single crystal superalloys which makes use of modelled composition-microstructure-property relationships. In particular, calculations are shown for the Ni-Cr-Co-Re-W-Al-Ta system in which data are plotted for various predicted characteristics of around 100,000 materials in the compositional space under consideration. By cycling over this wide compositional space, and eliminating from it materials which are deemed to be unsuitable it is possible to identify a number of prototype alloys for future testing.

A problem with this approach is that there are inherent uncertainties in the models used to make the alloy property predictions. For example, the uncertainty in a model varies as a function of multi-dimensional design space, with the uncertainty being higher in regions of extrapolation. Nonetheless, for many design scenarios a new material is being sought that has an optimal balance of properties for a given application, and which is an incremental, rather than a step-change, improvement over known materials. Material optimisation is therefore likely to seek sets of design variables with better balances of properties within well-characterised design space. Therefore, the models can mainly be used to interpolate, rather than to extrapolate.

However, significant sources of uncertainty still exist such as: the experimental input data used for the models, and the property model fitting process. Thus, in general, if a predicted property matches a target property there is a 50% probability that the real-life property will actually exceed the target property. It follows that if there are ten properties for which targets need to be satisfied, and if the predicted property matches the target property for each of them, then in the real-life material there is only a $0.5^{10} \approx 0.001$ probability that all of the target properties have been matched or exceeded.

Summary of the Invention

Material optimisation approaches that do not take account of the uncertainty in the model properties are at a significant disadvantage.

Accordingly, in a first aspect, the present invention provides a method of designing a material by optimising values for a plurality of design variables, the method including the steps of:

(i) providing one or more property models, the $i^{th}$ property model (where $i$ is an integer from 1 to $n_{prop}$, the number of property models) producing a prediction $P_i$ for a value of a respective property of the material as a function of the design variables, and further producing a value for the uncertainty $\sigma_i$ in the prediction $P_i$,
(ii) for each property, setting a specification target $T_{0,i}$ for a desired value of the property and a probability $p_i$ for that specification target $T_{0,i}$ to be met or exceeded,
(iii) for each property, determining a probabilistic target $T_i$ for a value of the property, the probabilistic target $T_i$ being based on the specification target $T_{0,i}$ and the probability $p_i$, and further defining a merit index factor $G_i$ based on the degree to which a given prediction $P_i$ satisfies the probabilistic target $T_i$,
(iv) constructing an overall merit factor G from the individual merit index factors $G_i$ of the properties, and
(v) determining a set of optimal design variables that optimise the overall merit factor G.

Each of the property models produces a prediction $P_i$ along with an uncertainty $\sigma_i$ in the prediction as a function of design space. Since the uncertainty generally varies as a function of design space, it is not appropriate to just set a constant specification target, since the probability of the exceeding that target will vary. For speculative areas of design space it is likely that the uncertainty on many of the predictions will be higher than for regions of design space that have been well investigated. However, by specifying the probability $p_i$ that a target is met or exceeded, the probabilistic target $T_i$, which will vary as a function of design space, can be determined, allowing regions with high uncertainty to be avoided in favour of more certain regions.

A second aspect of the present invention provides a method of producing a material including:

performing the method of the first aspect to identify a material having optimised values of the plurality of design variables in order to meet or exceed material property specification targets, and
preparing the material.

The method of the second aspect may further include testing the prepared material to determine whether its material properties meet or exceed the specification targets.

Further aspects of the present invention provide: a computer program comprising code which, when run on a computer, causes the computer to perform the method of the first aspect; a computer readable medium storing a computer program comprising code which, when run on a computer, causes the computer to perform the method of the first aspect; and a computer system programmed to perform the method of the first aspect.

For example, a computer system can be provided for designing a material by optimising values for a plurality of design variables, the system including one or more processing cores configured to: (i) provide one or more property models, the $i^{th}$ property model (where $i$ is an integer from 1 to $n_{prop}$, the number of property models) producing a prediction $P_i$ for a value of a respective property of the material as a function of the design variables, and further producing a value for the uncertainty $\sigma_i$ in the prediction $P_i$, (ii) for each property, set a specification target $T_{0,i}$ for a desired value of the property and a probability $p_i$ for that specification target $T_{0,i}$ to be met or exceeded, (iii) for each property, determine a probabilistic target $T_i$ for a value of the property, the probabilistic target $T_i$ being based on the specification target $T_{0,i}$ and the probability $p_i$, and further defining a merit index factor $G_i$ based on the degree to which a given prediction $P_i$ satisfies the probabilistic target $T_i$, (iv) construct an overall merit factor G from the individual merit index factors $G_i$ of the properties, and (v) determine a set of optimal design variables that optimise the overall merit factor $G$. The system thus corresponds to the method of the first aspect. The system may further include: a computer-readable medium or media operatively connected to the processing cores, the medium or media storing the property models. The system may further include: a display device for displaying the results of the optimisation and/or for setting the specification targets.

Optional features of the invention will now be set out. These are applicable singly or in any combination with any aspect of the invention.

The type of material that can be optimised is not limited as long as the property models exist. Typically, however, the material can be a metal alloy, such as a superalloy.

The design variables may include relative amounts of constituent elements of the material (i.e. relative amounts of alloying elements in the case of an alloy). There may be two or more, or five or more, and preferably ten or more, or twenty or more such elements.

The design variables may include values of processing conditions of the material, such as heat treatment temperature(s) and heat treatment duration(s).

A plurality of property models may be provided. For example, two or more, or five or more properties may be provided, or preferably ten or more, or twenty or more property models may be provided.

The property models may include neural network models. Advantageously, such models generally provide fast predictions of material properties. Also neural network models are suitable for providing values for the uncertainty in their predictions. Furthermore, neural network models may be used to interpolate for more computationally expensive models (such as e.g. CALPHAD models). Advantageously, neural network machine learning of more computationally expensive models can be amenable to automation. However, recourse to a more computationally expensive calculation of a property may be desirable if a neural network prediction has a greater effect on the overall merit factor than the sum of all of the merit index factors for the other properties.

The property models may include one or more mechanical property models, physical property models, *ab-initio* models (e.g. models that use underlying behaviour of electrons to calculate material properties - e.g. using density functional theory), phase diagram (e.g. CALPHAD) models and/or any other model that describes a material's behaviour.

Each merit index factor $G_i$ may take a substantially constant optimal value (e.g. zero) whenever the given prediction $P_i$ meets or exceeds the probabilistic target $T_i$. Thus, the merit index factor is generally flat so that the optimisation is not placed under further bias when the probabilistic target is satisfied. However, a slight slope may be placed upon selected individual merit index factors, e.g. when their respective predictions strongly exceed their probabilistic targets. This can then facilitate the optimisation of other properties whilst still satisfying all the probabilistic targets.

Conveniently, in step (v), the set of optimal design variables may be determined by performing a multi-variable optimisation based upon simulated annealing.

In step (v), the set of optimal design variables may be determined by performing a multi-variable optimisation in which a value of each design variable is adjusted by a respective step length, the size of each step length being adjustable to improve the search efficiency of the optimisation. For example, in "flatter" regions of overall merit index space, the step sizes may be increased, and in "steeper" regions, the step size may be reduced.

The method may include a further step of: (vi) determining the design variables which define the boundary of the region of multi-dimensional design variable space which includes the set of optimal design variables and which, for each property, produces a prediction $P_i$ which meets or exceeds the respective specification target $T_{0,i}$. For example, the determination may be accomplished by performing an acclivous search, in which the merit index factor for each property is adjusted in turn to $\xi G_i$, and further optimisation of the design variables is performed, $\xi$ biasing each merit index factor by an amount that is insufficient to push the other properties below their probabilistic targets $T_i$. The method may also include a further step of: (vii) identifying a set of design variables within said region of multi-dimensional design variable space which is most likely to produce predictions $P_i$ which meet or exceed the specification targets $T_{0,i}$. Within the region there may be a set of design variables which provide better properties than those determined at step (v), and further step (vii) can allow that set to be found.

This can be achieved by adjusting the merit index factor for each design property $\xi G_i$ in turn, where $\xi \approx 10^{-3}$ is an aggression factor and is chosen to be small so that the bias introduced on any individual merit factor is not sufficient to push other properties below their probabilistic targets $T_i$. The use of the adaptive searching technique automatically adopts to the new slope and allows efficient exploration of the optimum value for each property whilst retaining all other properties to be above their probabilistic targets

The term "computer readable medium" may represent one or more devices for storing data, including read only memory (ROM), random access memory (RAM), magnetic RAM, core memory, magnetic disk storage mediums, optical storage mediums, flash memory devices and/or other machine readable mediums for storing information. The term "computer-readable medium" includes, but is not limited to portable or fixed storage devices, optical storage devices, wireless channels and various other mediums capable of storing, containing or carrying instruction(s) and/or data.

Furthermore, embodiments may be implemented by hardware, software, firmware, middleware, microcode, hardware description languages, or any combination thereof. When implemented in software, firmware, middleware or microcode, the program code or code segments to perform the necessary tasks may be stored in a machine readable medium such as storage medium. A processing core(s) may perform the necessary tasks. A code segment may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

Further optional features of the invention are set out below.

Brief Description of the Drawings

Embodiments of the invention will now be described by way of example with reference to the accompanying drawings in which:

Figure 1 is a schematic illustration of overall merit factor global optimisation to find optimal design space;

Figure 2 is a probability distribution *f(Property)* for the probabilistic target $T_i$ in relation to the specification target $T_{0,I}$;

Figure 3 is a flow diagram showing the architecture of an embodiment of the optimisation approach; and

Figure 4 shows a merit index function for defining minimum properties.

Detailed Description and Further Optional Features of the Invention

The present invention provides a probabilistic and automated approach to material (e.g. alloy) optimisation. The optimisation can find, within a design space encompassing all possible composition and processing conditions (design variables), a composition and set of processing conditions that are predicted to produce a material capable of exceeding, by a user-defined probability, a set of material property specification targets. Once an optimal set of design variables is found, an acclivous search can then be conducted to explore the boundaries of multi-dimensional design space which satisfy the specification targets.

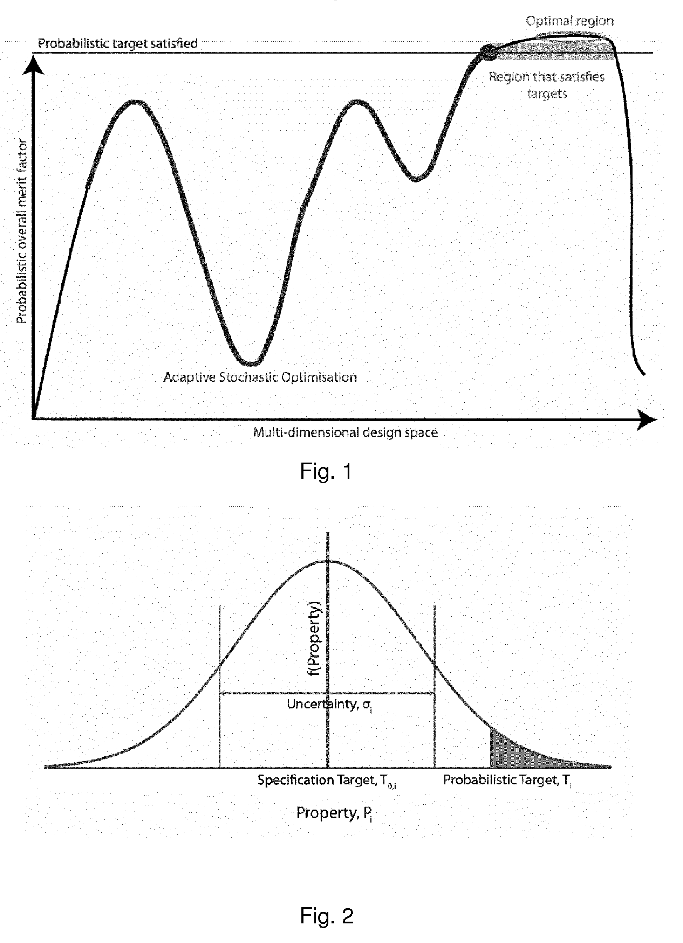

A schematic illustration of the approach is shown in Figure 1, whereby a single overall description of the material's likelihood of exceeding the specification targets, the "probabilistic overall merit factor", is constructed as a function of multi-dimensional design space, taking into account uncertainty in property models, and then optimised using an efficient global optimisation method. When a region has been found which satisfies the specification targets to the user-defined probability, the extent of the region can be determined, and the set of design variables that has the highest overall probability of exceeding the specification targets can be found.

A more detailed description of the approach follows and, for convenience, is divided into sections on: property acquisition from property models, selection of property specification targets and probabilities, optimisation of design variables, search of optimal design space region. Following this is a brief discussion of: computational speed issues, results of an example optimisation performed based on the commercial alloy "Astroloy", and a summary of advantages associated with the approach.

*Property Acquisition from Property Models*

The properties of a material are a function of its composition (i.e. relative amounts of constituent elements) and processing conditions, together these being termed the "design variables". A particular property can either be measured in an experiment from a representative sample of the material or estimated through computer modelling using the design variables as an input along with any required static variables (such as test temperature, test frequency or strain rate). Table 1 lists a number of property models that have been integrated into the material optimisation system. The majority of the neural network property models were constructed using publicly available data. The CALPHAD simulations were made using the commercially available Thermo-Calc™ software.

The use of neural networks to predict materials properties is well known, and described for example in H. K. D. H. Bhadeshia, R. C. Dimitriu, S. Forsik, J. H. Pak and J. H. Ryu, Performance of neural networks in materials science, Materials Science and Technology, 25, 5004 (2009). The number of hidden neurons was selected using sensitivity calculations to determine the effect of adding and removing hidden neurons and variables, the aim being to achieve the simplest property model possible that is an accurate reflection of the dataset to minimise the probability of overfitting.

The neural network models have defined boundaries, which limits the range within which the material optimization system can search. The data underpinning the neural network can be non-uniformly distributed over design space so there will be some regions of the multi-dimensional design space which are better described than others. To avoid poorly defined regions, an uncertainty function is employed to describe the accuracy of the model. For all of the neural network models, uncertainty can thus be predicted as a function of any combination of design variables and then used to set probabilistic targets, as described below.

Table 1

| Property | Major applications | Type of model | No. datapoints (where applicable) |
|---|---|---|---|
| (specific) Ultimate tensile stress | Ni γ/γ' alloy | Neural network | 2114 |
| (specific) Yield stress | Ni γ/γ' alloy | Neural network | 2248 |
| (specific) Stress rupture life | Ni γ/γ' alloy | Neural network | 2088 |
| Elongation | Ni γ/γ' alloy | Neural network | 1087 |
| Total stable γ + γ' mol.% fraction | Ni γ/γ' alloy | CALPHAD | - |
| Stable γ' mol.% fraction | Ni γ/γ' alloy | CALPHAD | - |
| Single phase window | Ni γ/γ' alloy | CALPHAD | - |
| Weldability (prevention of hot cracking) | Ni γ/γ' alloy | CALPHAD | - |
| Elemental cost | Any alloy | Rule of mixtures | - |
| Density | Any alloy | Rule of mixtures | - |
| Unconstrained lattice misfit | Ni γ/γ' alloy | CALPHAD, Vegard Law | - |
| Total γ phase amount | Ni γ single phase | CALPHAD | - |
| Creep model | Ni γ/γ' alloy | Neural network | 4948 |
| Low cycle fatigue | Ni γ/γ' alloy | Neural network | 15115 |
| High cycle fatigue | Ni γ/γ' alloy | Neural network | 3102 |
| Total stable γ + γ' mol.% fraction | Ni γ/γ' alloy | Auto Learning Neural network/CALPHAD | 37082 |
| Total γ phase amount | Ni γ single phase | Auto Learning Neural network/CALPHAD | 123451 |
| Oxidation | Ni γ/γ' alloy | Neural network | 315 |
| Stable γ' mol.% fraction | Ni γ/γ' alloy | Auto Learning Neural network/CALPHAD | 54895 |
| Stable γ' α mol.% | Ni γ' α (Pt) alloy | CALPHAD | - |
| γ' yield stress | Alloy with γ' | Neural network | 847 |
| α(Pt) ultimate tensile strength | Alloy with α (Pt) | Neural network | 740 |
| Stress rupture life | γ' α Ni-Al-Pt alloy | Neural network | 82 |
| Oxidation | γ' α Ni-Al-Pt alloy | Neural network | 821 |
| γ' solvus | γ/γ' alloy | CALPHAD | - |
| Molecular dynamics energy | any alloy | Auto learning neural network/MD simulator | - |
| Molecular dynamics lattice misfit | any alloy | Auto learning neural network/MD simulator | - |
| Minimum protective scale formers targets | Ni alloy | Minimum targets | - |

*Selection of Property Specification Targets and Probabilities*

To enable the optimisation of materials, the specification targets for the various properties which are to be met or exceeded in the optimised material are set. The selection of these targets is important; if over-ambitious targets are set, then the search for an optimal set of design variables is likely to be unsuccessful. To increase the likelihood that the optimisation results in the identification of a successful material, probabilities that the specification targets are met or exceeded are also set and used to determine probabilistic targets.

More particularly, since the property models are generated using incoherent experimental data gathered from a population of samples, the central limit theorem can be applied. The central limit theorem states that if x is a sequence of n independent and identically distributed random variables, each having mean $\mu$ and variance $\sigma^2$, then:

$$1/\sqrt{n} \sum_{n}^{k=1} (x_k - \mu)^2 \rightarrow N\left(\mu, \sigma^2\right) \tag{1}$$

Therefore, the uncertainty of predictions can be assumed to obey a normal distribution $N(\mu, \sigma^2)$. Using the normal distribution, a probabilistic target can be set which has a fixed probability, p, of exceeding the specification target. This is illustrated in Figure 2 by a Gaussian curve, *f(Property)*, which describes the probability distribution for a property model. The probabilistic target, $T_i$, for a particular property prediction, $P_i$, is given by the probit function,

$$T_i = T_{0,i} + \sqrt{2}\sigma_i \mathrm{erf}^{-1}\left(2p_i - 1\right) \tag{2}$$

where i is an integer which identifies each different property, $p_i$, is the probability defined for exceeding the given specification target, and $\sigma_i$ is the standard deviation for the property for a particular set of design variables.

Using a power series to represent the inverse error function erf$^{-1}$ for computational purposes with coefficients $i_{\mathrm{erf}}$ and taking into account that some properties are maximum specification targets whilst others are minimum specification targets, the probabilistic target $T_i$ for a property i is given by:

$$T_i = T_{0,i} + \sqrt{2}M_i\sigma_i \sum_{n=0}^{n_{limit}} i_{\mathrm{erf}_n} \left(\frac{\sqrt{\pi}}{2}(2p_i - 1)\right)^n \tag{3}$$

where $T_{0,l}$ is the specification target of a given property, $M_i$, takes the value of +1 if the target is a minimum specification target or -1 if the target is a maximum specification target, and $n_{\mathrm{limit}}$, is the limit of the power series. In addition to using probability $p_i$ to set probabilistic targets, the probability $p_c$ that any particular modelled property i will exceed its specification target is

$$p_c = 0.5 + 0.5M_i\mathrm{erf}\left(\frac{(P_i - T_i)}{\sqrt{2}\sigma_i}\right) \tag{4}$$

If it is assumed that all the material properties are independent, the probabilities $p_i$ can multiplied together to give an overall estimate for the probability that a set of design variables will exceed all the defined properties.

The use of probabilistic targets is generally sufficient to prevent searching within parts of multi-dimensional design space where extreme extrapolation of the property models occurs. However as an additional safeguard, the property models that are being searched can each have a range over which they are valid. This is of particular helpful when using a Thermo-Calc™ property model for which a nominal probability was defined which does not vary as a function of design space. The range can also used to set each design variable's initial step length for the optimisation (discussed below).

*Optimisation of Design Variables*

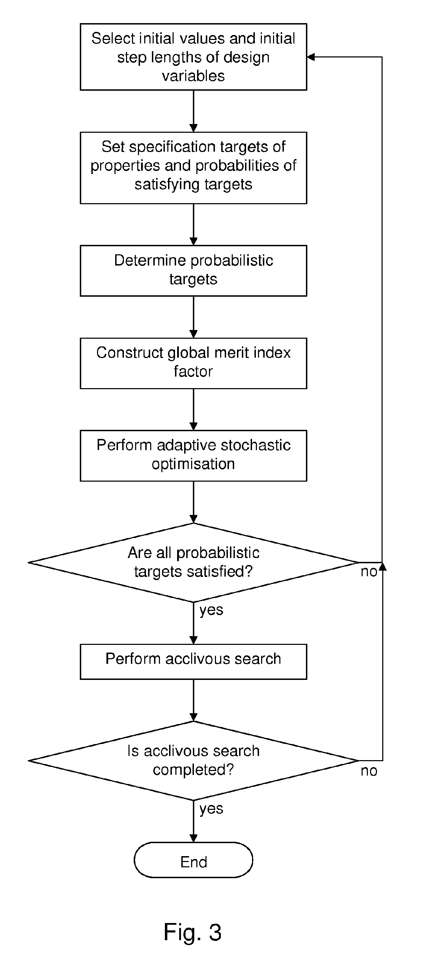

Figure 3 is a flow diagram showing the architecture of an embodiment of the optimisation approach.

A first stage is to set the initial values of the design variables of the material. For example, these can be based on the design variables of a known material having properties close to those targeted. Further, the initial step length in each design variable used for searching design space can be set, or a default value can be set. For example, the initial step length can be, for example, a fraction of the range of each design variable.

Next, specification targets $T_{0,i}$ are set for the properties, and probabilities $p_i$ for meeting or exceeding those targets are also set, as determined by the user. Modelling of the physical demands of an actual component would be a suitable way of determining the specification targets. Alternatively, they can be determined by adjusting the properties of a known (e.g. commercial) material.

The probabilistic targets $T_i$ can then be determined, e.g. using equation (3).

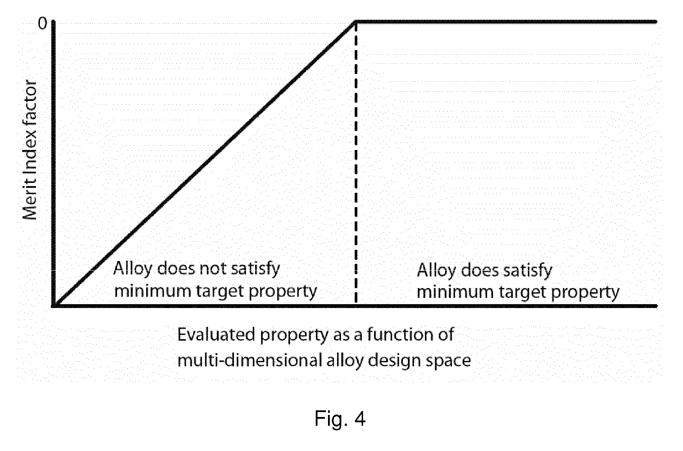

For each probabilistic target $T_i$ that the material is required to satisfy, each property $P_i$ is modelled as a function of multi-dimensional design space. Figure 4 shows a curve for a respective property $P_i$ which returns a merit index factor $G_i$ based on whether a particular combination of composition and processing conditions (design variables) satisfies the probabilistic target $T_i$. $G_i$ can, for example, be defined by equations (5) and (6) below in which, when $P_i < T_i$:

$$G_i = \frac{(T_i - P_i)M_i}{T_i - P_{min,i}} \qquad \text{for } P_i M_i < T_i \tag{5}$$

but otherwise:

$$G_i = 0 \tag{6}$$

where, $P_{min,i}$ is the minimum property value that a property model can take and $M_i$ takes the value of +1 if the target is a minimum specification target or -1 if the target is a maximum specification target. Figure 4 shows that the merit index factor has two regimes. When the property satisfies the target, then the merit function is flat so that the optimisation is not placed under further bias. When the property does not satisfy the target, a linear sloped section reduces the computational workload of the adaptive optimisation by reducing the automated alteration required for the optimisation parameters.

An overall merit factor is then constructed from the sum of all the individual merit index factors for each property as shown in equation (7).

$$G = \sum_{i=1}^{n_{prop}} G_i \tag{7}$$

where $n_{prop}$ is the number of properties being optimised. A summation can be used rather than any other means of combining the merit indices so that the partial derivatives of the combined merit indices with respect to any designed variable remain well behaved.

To accelerate the search, properties that strongly exceed their probabilistic targets can initially have a negative slope $\theta G_i$ imposed on their merit index factors to deliberately sacrifice their values in favour of properties that fall below their probabilistic targets. For example, $\theta$ may be about 1.

Thereafter, the overall merit factor G is maximised using an automated optimisation approach. This can be based on the well-known "simulated annealing" technique (which to avoid confusion with actual annealing of the subject materials, we prefer to term "adaptive stochastic optimisation"). The current combination of design variables and the overall merit factor are stored. Then a new combination of design space variables is chosen by random steps. As mentioned above the step length $s_j$ can initially be set equal to a fraction of the range of each design variable, which is approximately equal to the accuracy that the design variable can be experimentally evaluated. The proposed new design variable $x_j$ for each $j$ can be set in the following way:

$$x_{j,new} = x_{j,old} + (RAND - 0.5)\, s_j \qquad (8)$$

where $j$ represents each design variable and RAND is a random number between unity and zero.

The property values are calculated for each model, each returning the value $P_i$ for this new set of design variables and the merit index factor is evaluated for each model, the overall merit factor being the sum of all the individual merit index factors. If this overall merit factor is greater than the previous overall merit factor then the step is always accepted. If the overall merit factor is less than the previous overall merit factor then equation (9) can determine the acceptance likelihood $E$ for a transition, where $G_{old}$ is the previous overall merit factor, and G is the overall merit factor of the proposed transition. If $E$ is greater than a random number between 0 and 1 then the step is accepted, otherwise it is rejected. This process is repeated until the merit index factor for each property is equal to zero or until a user defined number of iterations is completed. Typically 500-1000 iterations are required to find a set of design variables which satisfy all the targets.

$$E = \exp\left((G - G_{old})/A\right) \qquad (9)$$

The acceptance factor $A$ can be automatically chosen to optimise the searching efficiency. This can be been determined as the optimal fraction of jumps that should be accepted to ensure all local minima can be successfully explored. The acceptance factor can be readjusted if the average acceptance rate is further than a standard deviation $\sigma_i$ away from the optimal acceptance rate of 0.352 (A. Gelman, G.O. Roberts, and W.R. Gilks, *Bayesian Statistics.* 5, 599 (1996)). This allows for rapid adjustment according changes in the combination of functions being evaluated, but retains acceptance factor stability with minimisation oscillation in the average number of steps accepted. Thus if $\overline{E} - \sigma_{\overline{E}} > 0.352$ then

$$A := \alpha A \qquad (10)$$

or if $\overline{E} + \sigma_{\overline{E}} < 0.352$

$$A := \frac{A}{\alpha} \qquad (11)$$

where $\alpha \approx 0{:}8$ is a response factor set according to the optimisation behaviour, and := is an assignment operator.

In addition to adjusting the acceptance factor, the step length can be automatically adjusted for each design variable to optimise the exploration of merit space based upon the average length of each accepted step. This is to optimise the search efficiency, so that in say "flatter" regions of merit index space, the step size is increased, and in "steep" regions or regions where only a small fraction of proposed steps are being accepted, the step size is reduced. Assuming a uniform probability distribution well describes the number of accepted steps, the step size can be adjusted in the following way.

An upper permitted limit $U_{LIM,J}$ and a lower permitted limit $L_{LIM,j}$ are calculated from the sum of the moving average accepted step length for each design variable $\overline{|a_j|}$ and the standard deviation of these average accepted design variables $\alpha_{aj}$.

$$U_{LIM,j} = \overline{|a_j|} + \sigma_{a_j},\; L_{LIM,j} = \overline{|a_j|} + \sigma_{a_j} \qquad (12)$$

The distribution of accepted step lengths is assumed to correspond to a uniform probability distribution, since the actual proposed step is a random number multiplied by a maximum possible step length $s_j$ as illustrated in equation (8). If all steps are accepted, the expected fraction within these bands is given by

$$\int_{z=0}^{0.5} z\, dz = \frac{1}{4}$$

(13)

Therefore, the current step length $s_j$ should not be greater or less than a quarter of the average accepted step length within a standard deviation. So if

$$\frac{s_j}{4} > U_{LIM,i}$$

then,

$$s_j = 4\overline{|a_j|}$$

(14)

or if $L_{LIM,i} < \frac{s_j}{4}$,

$$s_j := \beta s_{j1}$$

(15)

where $\beta \approx 2$, is a constant factor for which the step size $s_j$ should be increased to speed up the optimisation process, if calculated to be too small.

If, after the user defined number of iterations is completed, the merit index for each property does not equal to zero, the process can be recommenced with different initial values of the design variables of the material can be attempted, and/or different specification targets, or it may be concluded that no suitable material can be developed.

*Search of Optimal Design Space Region*

Once a combination of design variables which is predicted to meet or exceed the set of specification targets by the user stated probability is identified, an acclivous search can be conducted to search for the range of each property whilst simultaneously satisfying all the other property targets. This can be achieved by adjusting the merit index factor for each property in turn to $\xi G_i$, where $\xi \approx 10^{-3}$ is an aggression factor and is chosen to be small so that the bias introduced on any individual merit index factor is not sufficient to push other properties below their probabilistic targets $T_i$. The use of the adaptive searching technique automatically adopts to the new slope and allows efficient exploration of the optimum value for each property whilst retaining all other properties to be above their probabilistic targets.

Similarly to an unsuccessful optimisation, if the acclivous search does not proceed due to impossible sets of probabilistic property targets, the process can be recommenced with different initial values of the design variables of the material, and/or different specification targets.

*Computational Speed*

The rate-determining step in the multi-dimensional sampling process is generally the rate at which properties can be evaluated. For models such as neural networks or analytical calculations, evaluation of a property takes a very small fraction of a second. For more computationally expensive models, property evaluation can take anything from a few seconds to several hours. If thousands of property evaluations are required, then the overall optimisation process will be impractically slow.

Thus to increase the speed of the optimization process, neural network models can be used to replace more computationally expensive models, such as Thermo-Calc™. However, at some point during the optimization process, the neural network may no longer be able to accurately describe the data set because extreme extrapolation will be required to obtain the property in question. This can be determined to be the case if the uncertainty on any property has a greater effect on the overall merit index than all the other merit index factors added together. When this occurs, a property calculation can be activated for that particular set of design variables using the more computationally expensive model, and the result of the new property calculation added to the neural network data-set. The neural network can then be automatically retrained, and used to evaluate the design space surrounding the new point that has been calculated.

Parallelisation can also be adopted to make use of processors with multiple cores and hyper-threading, and which can execute some operations (such as property calculations) simultaneously. By use of parallelisation and hyper-threading, threads can exchange information with other threads and dramatically reduce the number of serial iterations that need to be performed.

*Example Optimisation*

The commercial alloy "Astroloy" has the wt% composition and is subjected to the heat treatments set out in Table 2. It also has the predicted properties set out in Table 3.

Table 2

| Astroloy | wt.% |
|---|---|
| Ni | 55.430 |
| Co | 16.800 |
| Cr | 14.600 |
| Mo | 5.200 |
| Al | 4.100 |
| Ti | 3.540 |
| Fe | 0.250 |
| C | 0.035 |
| Si | 0.020 |
| B | 0.000 |
| Cu | 0.000 |
| Hf | 0.000 |
| Mn | 0.000 |
| N | 0.000 |
| Nb | 0.000 |
| P | 0.000 |
| Ta | 0.000 |
| V | 0.000 |
| W | 0.000 |
| Zr | 0.000 |
| Heat treatment 1 temperature /°C | 1200 |
| Heat treatment 1 duration /hrs | 2 |
| Heat treatment 2 temperature /°C | 800 |
| Heat treatment 2 duration /hrs | 8 |

Table 3

| Property | Prediction | Uncertainty |
|---|---|---|
| Cost /$lb$^{-1}$ | 10.3 | 0.00 |
| Density /kgm$^{-3}$ | 8070 | 0.00 |

(continued)

| Property | Prediction | Uncertainty |
|---|---|---|
| Low Cycle Fatigue $10^x$ cycles | 4.95 | 1.0 |
| High Cycle Fatigue $10^x$ cycles | 6.40 | 1.0 |
| (specific) Ultimate Tensile Stress /MPakg$^{-1}$m$^3$ | 0.139 | 0.016 |
| (specific) Yield Stress /MPakg$^{-1}$m$^3$ | 0.095 | 0.013 |
| (specific) Stress Rupture /MPakg$^{-1}$m$^3$ | 0.084 | 0.014 |
| (specific) Elongation /%kg$^{-1}$m$^3$ | 0.002 | 0.001 |

By way of an example, the optimisation approach descried above was used to search for an alloy in which the ultimate tensile strength was raised from 0.139 MPakg$^{-1}$m$^3$ to 0.150 MPakg$^{-1}$m$^3$. All the other specification targets were set to Astroloy's properties, the heat treatment was fixed, and the composition was allowed to vary. After running the program for 5000 "adaptive stochastic optimisation" iteration cycles, it was predicted that an alloy having the composition set out in Table 4 would have the properties set out in Table 5, thereby satisfying the specification targets including the enhanced ultimate tensile strength.

Table 4

| "Improved" alloy | wt.% |
|---|---|
| Ni | 41.049 |
| Co | 18.991 |
| Cr | 18.249 |
| Mo | 4.367 |
| Al | 4.021 |
| Ti | 4.310 |
| Fe | 8.324 |
| C | 0.003 |
| Si | 0.033 |
| B | 0.033 |
| Cu | 0.000 |
| Hf | 0.000 |
| Mn | 0.001 |
| N | 0.000 |
| Nb | 0.101 |
| P | 0.000 |
| Ta | 0.470 |
| V | 0.000 |
| W | 0.036 |
| Zr | 0.012 |
| Heat treatment 1 temperature /°C | 1200 |
| Heat treatment 1 duration /hrs | 2 |
| Heat treatment 2 temperature /°C | 800 |
| Heat treatment 2 duration /hrs | 8 |

Table 5

| Property | Prediction | Uncertainty |
|---|---|---|
| Cost /$lb$^{-1}$ | 9.95 | 0.0 |
| Density /kgm$^{-3}$ | 7900 | 0.0 |
| Low Cycle Fatigue $10^x$ cycles | 4.96 | 1.0 |
| High Cycle Fatigue $10^x$ cycles | 9.90 | 1.0 |
| (specific) Ultimate Tensile Stress /MPakg$^{-1}$m$^3$ | 0.177 | 0.016 |
| (specific) Yield Stress /MPakg$^{-1}$m$^3$ | 0.150 | 0.013 |
| (specific) Stress Rupture /MPakg$^{-1}$m$^3$ | 0.096 | 0.014 |
| (specific) Elongation /%kg$^{-1}$m$^3$ | 0.002 | 0.001 |

*Summary*

Significant advantages of this approach to material optimisation are:

• Optimisation of multiple properties of a material.

• Generation and use of property models that calculate uncertainties in their predictions, allowing the uncertainties in the predicted properties of the optimised material to be provided.

• Use of probabilistic targets that guide the optimisation to regions of design space where material properties are more certain, thereby increasing the likelihood that an optimised alloy will have the desired target properties.

• Amenability to techniques that can accelerate the optimisation.

To produce fast, robust, and accurate optimisations, amenable to automation, the approach can be implemented using:

• Adaptive stochastic optimisation, or alternatives such as (but not limited to) genetic algorithms, particle swarm, quantum stochastic optimisation, and multilevel coordinate search for which optimisation parameters are automatically determined.

• Machine learning (i.e. neural networks) to provide faster alternatives to computationally expensive models.

• Automated prediction of optimisation algorithm parameters.

• Acclivous searching to place boundaries on acceptable material compositions.

• Parallelisation of operations.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

All references referred to above are hereby incorporated by reference.

**Claims**

1. A method of designing a material by optimising values for a plurality of design variables, the method including the steps of:

(i) providing one or more property models, the $i^{th}$ property model (where i is an integer from 1 to $n_{prop}$, the number of property models) producing a prediction $P_i$ for a value of a respective property of the material as a function of the design variables, and further producing a value for the uncertainty $\sigma_i$ in the prediction $P_i$,

(ii) for each property, setting a specification target $T_{0,i}$ for a desired value of the property and a probability $p_i$ for that specification target $T_{0,i}$ to be met or exceeded,

(iii) for each property, determining a probabilistic target $T_i$ for a value of the property, the probabilistic target $T_i$ being based on the specification target $T_{0,i}$ and the probability $p_i$, and further defining a merit index factor $G_i$ based on the degree to which a given prediction $P_i$ satisfies the probabilistic target $T_i$,

(iv) constructing an overall merit factor G from the individual merit index factors $G_i$ of the properties, and

(v) determining a set of optimal design variables that optimise the overall merit factor G.

2. A method according to claim 1, wherein the design variables include relative amounts of constituent elements of the material.

3. A method according to claim 1 or 2, wherein the design variables include values of processing conditions of the material.

4. A method according to any one of the previous claims, wherein the property models include neural network models.

5. A method according to claim 4, wherein one or more of the neural network models interpolate for more computationally expensive models.

6. A method according to any one of the previous claims, wherein the property models include one or more mechanical property models, *ab-initio* models, physical property models and/or CALPHAD models.

7. A method according to any one of the previous claims, wherein each merit index factor $G_i$ takes a substantially constant optimal value whenever the given prediction $P_i$ meets or exceeds the probabilistic target $T_i$.

8. A method according to any one of the previous claims, wherein, in step (v), the set of optimal design variables are determined by performing a multi-variable optimisation based upon simulated annealing.

9. A method according to any one of the previous claims, wherein the method includes a further step of:

(vi) determining the design variables which define the boundary of the region of multi-dimensional design variable space which includes the set of optimal design variables and which, for each property, produces a prediction $P_i$ which meets or exceeds the respective specification target $T_{0,i}$.

10. A method according to claim 9, wherein the method includes a further step of:

(vii) identifying a set of design variables within said region of multi-dimensional design variable space which is most likely to produce predictions $P_i$ which meet or exceed the specification targets $T_{0,i}$.

11. A method of producing a material including:

performing the method of any one of the previous claims to identify a material having optimised values of the plurality of design variables in order to meet or exceed material property specification targets, and preparing the material.

12. A computer system programmed to perform the method of any one of claims 1 to 10.

13. A computer program comprising code which, when run on a computer, causes the computer to perform the method of any one of the claims 1 to 10.

14. A computer readable medium storing a computer program comprising code which, when run on a computer, causes the computer to perform the method of any one of the claims 1 to 10.

Fig. 1

Fig. 2

Fig. 3

Fig. 4

**EP 2 778 990 A2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **H. K. D. H. BHADESHIA ; R. C. DIMITRIU ; S. FORSIK ; J. H. PAK ; J. H. RYU.** Performance of neural networks in materials science. *Materials Science and Technology,* 2009, vol. 25, 5004